# EUROPEAN PATENT APPLICATION

(11) **EP 3 103 405 A1**
(43) Date of publication of application: **14.12.2016**
(21) Application number: 15002947.8
(22) Date of filing: 15.10.2015
(51) Int. Cl.: A61B 17/34

(54) **DEVICE FOR PROVIDING ACCESS INTO HEART INTERIOR**

(30) Priority: 08.06.2015 PL 41262915
(71) Applicant: Innovations for Heart and Vessels Sp. z o.o., 40-171 Katowice (PL)
(72) Inventor: Buszman, Piotr, 40-761 Katowice (PL); Buszman, Pawel, 40-748 Katowice (PL); Milewski, Krzysztof, 40-748 Katowice (PL)
(74) Representative: Gizinska-Schohe, Malgorzata

(57) **Abstract**

The invention relates to a device providing access to the interior of the heart, comprising at least one access channel, characterised in that it consists of two detachable parts: an outer part **(1)** comprising a cylindrical element **(5)** of a smaller diameter, provided inside with the access channel **(8)** designed to introduce medical devices to the interior of the heart and an outer flange **(2)** surrounding it externally at one end, located in a plane perpendicular to this element **(5),** and an inner part **(3)** comprising a cylindrical element **(6)** of a larger diameter and an inner flange **(4),** surrounding it, located in a plane perpendicular to this element **(6),** parallel to the outer flange **(2),** on the opposite side relative to this flange **(2),** wherein the cylindrical element **(5)** of the outer part **(1)** is located inside the cylindrical element **(6),** on one axis therewith, and their interconnection is ensured by a connecting latch **(7)** located on the plane of adhesion of the cylindrical elements **(5, 6).**

## Description

The object of the invention is a device providing access to an interior of a heart, i.e. a cardiological device (cardiological port), allowing introduction of other medical devices, through the heart wall, into the internal space of the heart, also with the heart beating. The device may provide a temporary or permanent access to the heart. The invention relates to the field of medical devices which minimise the invasiveness of subsequent cardiological procedures and diagnostic procedures.

Currently, many procedures on the heart are performed during complex surgeries on the open chest and with the heart action stopped. These procedures (particularly those performed again) are of high risk, are time-consuming and expensive, and the recovery period is prolonged. Progress in this field is possible thanks to, among other things, percutaneous interventions during which various types of medical devices are introduced into the heart area, through blood vessels, but such an approach is not always possible. Therefore, there is a continuing need to develop new methods which would minimise the invasiveness of cardiological procedures and diagnostic tests.

From European patent application EP2601897A1, a device providing access to the internal space of the heart is known which comprises a one-piece cylindrical element with two profiled elements surrounding the outer surface of the myocardium at the place where the cylindrical element is arranged.

From US patent application US2002161378A1, a device designed to conduct cardiological procedures on the beating heart is known which comprises a channel providing access to the interior of the heart, wherein the channel comprises at least one valve for opening and closing the channel. The device is adapted for transport of fluid between the channel and the space outside the heart. The device comprises a plug protecting the hole, for the time when it is not used.

Other types of devices providing access to the heart are also disclosed in the specifications of applications US2014148786A1 and US2005137609A1.

The aim of the present invention is to develop an improved device providing a facilitated, multiple (permanent) access to the interior of the heart. It is desirable to develop a universal device the channel of which allows introduction of various types of medical devices and smooth conduct of cardiological procedures.

The device providing access to the interior of the heart, comprising at least one access channel according to the invention consists of two separable parts: 1) an outer part comprising a cylindrical element of a smaller diameter, provided inside with the access channel designed to introduce medical devices to the interior of the heart and an outer flange surrounding it externally at one end, located in a plane perpendicular to this cylindrical element, and 2) an inner part comprising a cylindrical element of a larger diameter and an inner flange, surrounding it, located in a plane perpendicular to this cylindrical element, parallel to the outer flange, on the opposite side relative to this flange, wherein the cylindrical element of the outer part of the device is located inside the cylindrical element of the inner part of the device, on one axis therewith, and their interconnection is ensured by a connecting latch located on the plane of adhesion of the cylindrical elements, preferably consisting of opposite convex and concave profiles cooperating with each other.

The device is provided with a movable plug or a valve protecting the access channel.

The access channel may be provided inside with at least one sealing lip.

The outer flange and possibly the inner flange of the device are provided with mounting holes.

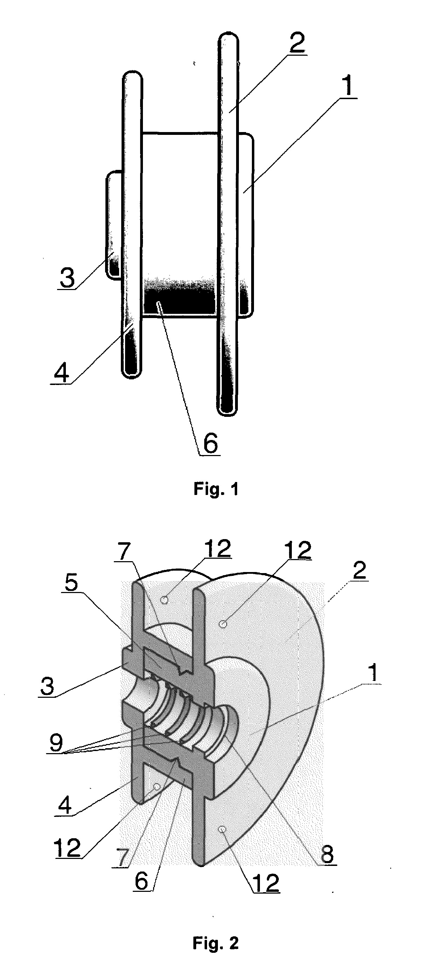

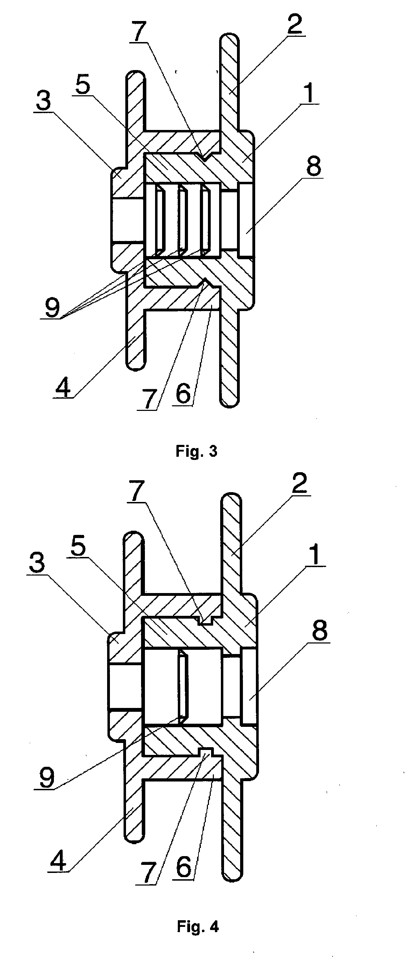

The object of the invention in an exemplary embodiment is illustrated in the drawing, in which Fig. 1 shows a spatial general side view of the device, Fig. 2 shows a spatial view of the device in cross-section, with the internal access channel exposed, Fig. 3 shows a cross-section of the device according to an embodiment with three sealing lips and the latch in a first variant, Fig. 4 shows a cross-section of the device according to an embodiment with one sealing lip and with the latch in a second variant, Fig. 5 shows a cross-section of the device with the plug disposed inside the access channel, and Fig. 6 shows a cross-section of the device in another embodiment with a valve protecting the access channel.

A device providing access of medical and/or diagnostic devices, to the interior of the heart, comprising at least one access channel, according to the invention, consists of two detachable parts, i.e. the first part - an outer part **1**, arranged, once implanted, on the outer side of the heart wall, comprising a cylindrical element **5** provided inside with a through access channel **8** designed to introduce medical devices to the interior of the heart through the heart wall, and an outer flange **2**, mounted thereon, surrounding it externally at one end, located in a plane perpendicular to this element **5**, and - the second part - an outer part **3** of the device (designed to be placed on the inner side of the heart wall) comprising a cylindrical element **6** of a larger diameter than the diameter of the cylindrical member **5** of the first part of the device, and an inner flange **4** surrounding it, located in a plane perpendicular to this element **6**, located in a plane parallel to the outer flange **2** of the first part of the device, on the opposite side relative to this flange **2**. The two parts of the device, once connected, ensure formation of the access channel passing through the heart wall, allowing communication between the external and internal parts of the heart.

The outer flange **2** of the device has a larger diameter than the inner flange **4**, but it is also possible that the diameters of both elements are similar. The flanges of the inner and/or outer part have a circular shape, but a different rounded shape not threatening tissue irritations and minimising the impact of the device on the mechanics of the heart is also possible. The cylindrical element **5** of the outer part **1** of the device is located inside the cylindrical element **6**, on one axis therewith, and their interconnection is ensured by a detachable connecting latch **7** located on the plane of adhesion of the cylindrical elements **5**, **6**, consisting of latching means cooperating with each other in the form of opposite convex and concave profiles which are mutually adapted with their shapes to achieve the effect of latching the two parts of the device. For example, when the convex profile on the wall of the cylindrical element has the cross-section of a wedge (triangle), the concave profile corresponding to it is a reflection thereof, i.e. is adapted to it in shape and is in the form of a triangular notch. The latching means used in the device according to the invention are designed so that their latching occurs by pressing one part of the device into the other. At the same time, it is possible to separate these parts by exerting an appropriate lateral pressure and/or prying the cylindrical elements of the device. The latch may be located all over the periphery of the cylindrical elements or only on portions of the periphery of the cylindrical elements.

The cylindrical elements **5**, **6** bf the device have a length adjusted to the thickness of the heart wall and may have different sizes depending on physiological conditions so that the space between two flanges mounting and stabilising the device is appropriately adjusted to the thickness of the heart wall.

The device according to the invention is optionally provided with an inserted and removed or screwed and unscrewed plug **10** or with a flexible valve **11** in the form of a membrane, protecting the access channel **8**. The plug **10** is a movable element and reveals/closes the access channel depending on the needs. The valve **11** is an element permanently arranged in the access channel and is flexible enough so it can be provided with a slot allowing the medical device to be forced through the valve. After removal of the device through the valve, it is tighten again.

The sealing lip **9** present inside the access channel is optionally placed in the device when it is provided with a plug. The lip **9** may be in the form of a separate seal in the shape of a rim, placed in the access channel **8**, or may be a flexible, convex profile extruded in a uniform wall of the access channel. In another embodiment of the invention, the sealing lip may also be in the form of several sections arranged on the inner periphery of the channel, and may also take the form of a spiral or several separate sections of a spiral. The sealing lips may be stabilised on a substrate, which is constituted by the wall of the channel, by means of an adhesive-sealed joint. In order to achieve tightness of the connection between the device according to the invention and the plug, one or more sealing lips may be used. In the case where the plug is in a screwable form, appropriately profiled screw threads on the side walls of the plug and of the channel will at the same time act as sealing lips. In other words, the sealing lips are in a spiral (threaded) form.

Furthermore, the outer flange may be provided with additional stabilising mounting holes **12** which either can be designed for the use of surgical threads which, once passed through the holes, will fasten the device to the myocardium, or these holes can be used for different types of screws carried through a portion of the myocardium in order to fasten the device. The mounting screws may be carried up to the surface of the inner flange and may provide stabilisation of the device on the myocardium by joining together the two flanges. The outer flange may comprise, for example, from two to eight, e.g. three, four or six, holes. Accordingly, the inner flange may also comprise from two to eight holes. When both flanges comprise holes, preferably these holes are approximately arranged oppositely so that stabilisation of the device is most effective.

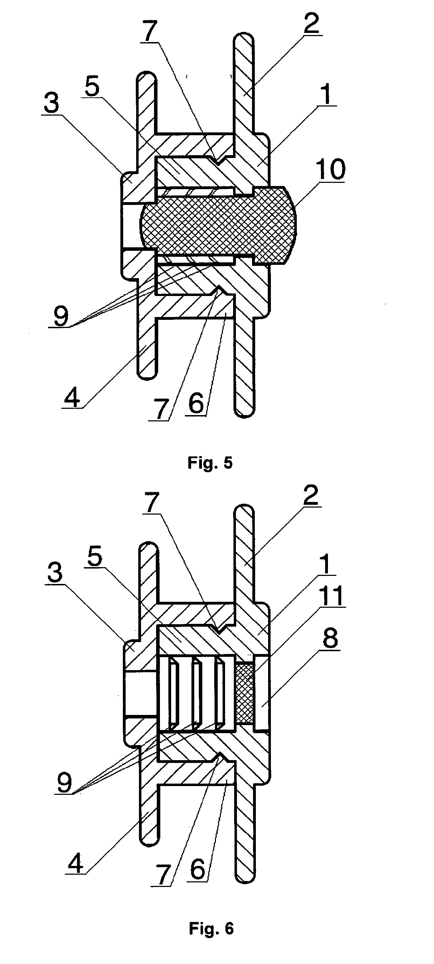

Exemplary embodiment of the invention, with the access channel protected by the plug, is shown in Fig. 5. In order to ensure a higher tightness of such a protection of the channel, it may be divided into sections of various diameters, i.e. on the section of the inner part of the device, the channel may have a smaller diameter ensuring a tight adherence of the plug end to the channel walls. On the middle section of the channel, its diameter may be slightly higher so that it is possible to place the sealing lips in the channel and to from a space for their correct positioning. On the section of the outer part, the access channel may, again, have a diameter smaller than on the middle section. In this way, the tightness of the closure of the access channel is protected at its three distinct sections. It is possible to use only one of the above protections, i.e. to use only narrowing of the channel or only the sealing lips, or only the plug.

Fig. 6 shows another embodiment of the invention, where the access channel is protected by a valve disposed in the narrowing of the access channel.

The device according to the invention can be made of various known materials, e.g. flexible Teflon, portions of titanium, stainless steel 316L, cobalt chromium alloy L605, possibly reinforced with a nitinol mesh.

References in the drawings:
1. outer part of the device
2. flange of the outer part of the device (outer flange)
3. inner part of the device
4. flange of the inner part of the device (inner flange)
5. cylindrical element of the outer part of the device
6. cylindrical element of the inner part of the device
7. connecting latch
8. access channel
9. sealing lip
10. plug
11. valve
12. mounting holes in the flange

## Claims

1. A device providing access to an interior of a heart, comprising at least one access channel, **characterised in that** it consists of two detachable parts:
- an outer part (**1**) comprising a cylindrical element (**5**) of a smaller diameter, provided inside with the access channel (**8**) designed to introduce medical devices to the interior of the heart and an outer flange (**2**) surrounding it externally at one end, located in a plane perpendicular to this element (**5**), and
- an inner part (**3**) comprising a cylindrical element (**6**) of a larger diameter and an inner flange (**4**), surrounding it, located in a plane perpendicular to this element (**6**), parallel to the outer flange (**2**), on the opposite side relative to this flange (**2**),
wherein the cylindrical element (**5**) of the outer part (**1**) is located inside the cylindrical element (**6**), on one axis therewith, and their interconnection is ensured by a connecting latch (**7**) located on the plane of adhesion of the elements (**5**, **6**).

2. The device according to claim 1, **characterised in that** the connecting latch (**7**) consists of opposite convex and concave profiles cooperating with each other.

3. The device according to claim 1 or 2, **characterised in that** it is provided with a movable plug (**10**) or with a flexible valve (11), protecting the access channel (**8**).

4. The device according to claim 1 or 2, or 3, **characterised in that** the access channel (**8**) is provided inside with at least one sealing lip (**9**).

5. The device according to claim 1 or 2, or 3, or 4, **characterised in that** the outer flange (**2**) and possibly the inner flange (**4**) are provided with mounting holes (**12**).
